# EUROPEAN PATENT APPLICATION

(11) **EP 0 719 858 A2**
(43) Date of publication of application: **03.07.1996**
(21) Application number: 95309476.0
(22) Date of filing: 27.12.1995
(51) Int. Cl.: C12N 5/10, C12N 15/12, C07K 14/72, G01N 33/567, G01N 33/564, C07K 16/28

(54) **Myeloma cell line expressing recombinant human thyroid stimulating hormone receptor**

(30) Priority: 27.12.1994 JP 325015/94
(71) Applicant: Tosoh Corporation, Shinnanyo-shi, Yamaguchi-ken, 746 (JP)
(72) Inventor: Matsuba, Takao, Atsugi-shi, Kanagawa (JP); Yasukawa, Kiyoshi, Sagamihara-shi, Kanagawa (JP)
(74) Representative: Kearney, Kevin David Nicholas

(57) **Abstract**

A myeloma cell line expressing recombinant human thyroid stimulating hormone receptor which may be efficiently cultured and may be easily collected to prepare a membrane fraction, and an immunochemical assay method for autoantibody against human thyroid stimulating hormone receptor which uses the membrane fraction from the cell line.

## Description

### BACKGROUND OF THE INVENTION

### 1. Field of the Invention

The present invention relates to a myeloma cell line expressing recombinant human thyroid stimulating hormone, to a method for producing a membrane fraction containing recombinant human thyroid stimulating hormone receptor, to an assay method for anti-human thyroid stimulating hormone receptor autoantibody and to a reagent kit for assaying anti-human thyroid stimulating hormone receptor autoantibody.

### 2. Related Art

Human thyroid stimulating hormone receptor (hereinafter, TSHR) is a receptor for thyroid stimulating hormone (TSH) which is present on the thyroid membrane, and when TSH secreted from the pituitary gland binds to TSHR on thyroid follicle cell membranes, the thyroid secretes the metabolism-regulating hormones T3 and T4. TSHR is a 7-fold membrane-spanning receptor of approximately 87,000 molecular weight, the extracellular domain of which has a molecular weight of about 45,000.

Basedow's disease is hyperthyroidism caused by accelerated production and secretion of thyroid hormone. One of its causes is the presence of stimulating substance in patients' serum, which promotes the secretion of thyroid hormone. Research to date has clearly demonstrated that autoantibodies against thyroid components are present in patient serum, and that these result in the production and secretion of thyroid stimulating hormone, which finally leads to deterioration of thyroid tissue.

Typical antigens used for the diagnosis of thyroid autoimmunity in Basedow's disease include thyroid peroxidase, thyroglobulin and TSHR, but it is known that anti-TSHR antibody is most strongly associated with abnormal thyroid functions accompanying the disease.

A presently known method for assaying anti-TSHR autoantibody is the method developed by Smith (Endocr. Rev., 9, 106-120, 1988), and reagents for the method are commercially available (Cosmic Co and others). In this method, porcine thyroid membrane fraction is used as a source of TSHR, and anti-TSHR autoantibody is detected by examining the competition against the TSHR source by ¹²⁵I-labeled bovine TSH and anti-TSHR autoantibody in the patient serum.

Since the conventional method makes use of cross-reaction binding of porcine TSHR with anti-human TSHR autoantibody in human serum, there have been doubts as to whether it accurately reflects binding of human TSHR intrincially produced in the body with anti-human TSHR autoantibody in human serum. Because the amino acid sequences of human TSHR and porcine TSHR differ in actuality, it is reasonable to assume that the binding of porcine TSHR with anti-human TSHR autoantibody in the conventional method is not completely identical to binding of human TSHR with anti-human TSHR autoantibody. Nevertheless, the use of living human-derived thyroid membrane fraction instead of porcine thyroid membrane fraction as a source of TSHR is impossible in the light of ethical and other issues.

In 1989, the gene for human TSHR was isolated and preparation of Chinese hamster ovary cells (CHO cells) expressing recombinant TSHR was reported (Japanese Unexamined Patent Publication Nos. 5-504683, and 5-503849). Expression of about 10⁵ TSHR molecules was confirmed in the membranes of the human TSHR-expressing CHO cell lines described in these documents, which was notably higher than the expression of a few hundred per cell in the previously used porcine thyroid membrane fraction. These documents also describe assay methods using CHO cell membrane fractions as thyroid membrane fractions as well.

However, since CHO cells are adherent cells, they cannot be grown in suspended culture. Cultured adherent cells are generally recovered by treatment with a protease such as trypsin, but recovery of the above-mentioned CHO cells by protease treatment cleaves the TSHR in the membrane fraction and thus it is difficult to prepare a membrane fraction which can be used for binding to anti-human TSHR autoantibody.

On the other hand, although membrane fractions usable for binding to anti-human TSHR autoantibody can be prepared if the cells are recovered by mechanical methods, this is inefficient and does not meet the demands of the clinical field which requires large amounts of TSHR.

### SUMMARY OF THE INVENTION

In order to overcome the aforementioned disadvantages, the present inventors have conducted extensive research on expression of TSHR, etc. and, as a result, have established a myeloma cell line expressing recombinant human thyroid stimulating hormone receptor, which can be efficiently cultured and recovered for use in preparing a membrane fraction, and have also accomplished an immunochemical assay method for autoantibody against human thyroid stimulating hormone receptor which uses the membrane fraction from those cells.

In other words, the present invention relates to a myeloma cell line expressing recombinant TSHR.

The present invention further relates to a method for producing a membrane fraction containing recombinant TSHR which is characterized by culturing a myeloma cell line which expresses recombinant TSHR.

The present invention still further relates to a method for assaying anti-human TSHR autoantibody which comprises a step of contacting a specimen to be analyzed with a membrane fraction containing recombinant TSHR produced by culturing the myeloma cell line which expresses recombinant TSHR.

The present invention still further relates to a reagent kit for the assay of anti-human TSHR autoantibody, which includes at least a membrane fraction containing recombinant TSHR produced by culturing the myeloma cell line which expresses recombinant TSHR.

### BRIEF DESCRIPTION OF THE DRAWINGS

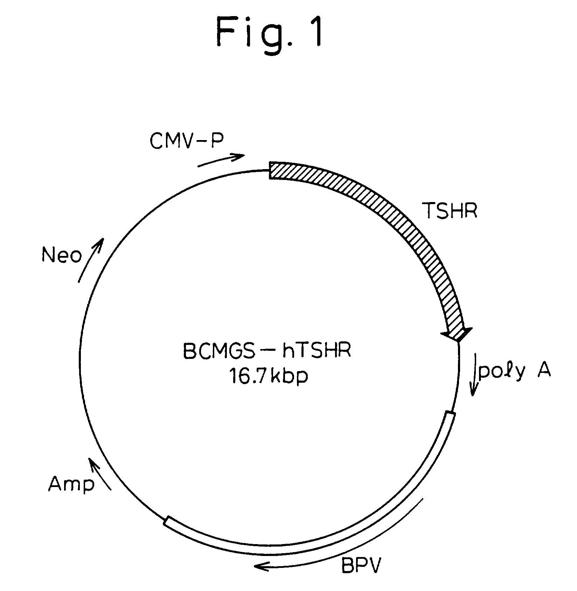

Fig. 1 is a sketch showing a membrane TSHR gene-expressing vector (BCMGS-hTSHR). In this sketch, CMV-P stands for cytomegalovirus promoter, TSHR, BPV and Amp stand for the genes encoding TSHR, bovine papilloma virus and β-lactamase, respectively, and poly A stands for polyadenylate attachment signal. Also, Neo stands for neomycin-resistance gene.

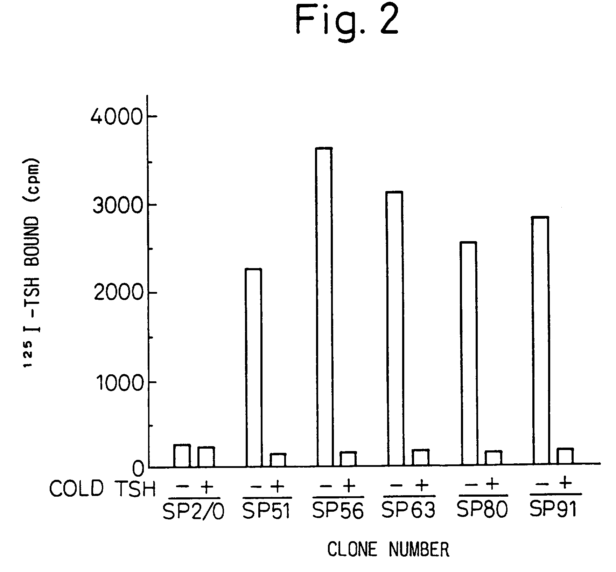

Fig. 2 is a graph showing a comparison of the binding activity of labeled TSH against 5 myeloma cell lines of the present invention (SP51, 56, 63, 80 and 91) expressing recombinant TSHR and against the conventional myeloma cell line SP2/0. In the graph, "+" denotes the amount of bound labeled TSH in the presence of unlabeled TSH, and "-" denotes the amount of bound labeled TSH in the absence of unlabeled TSH.

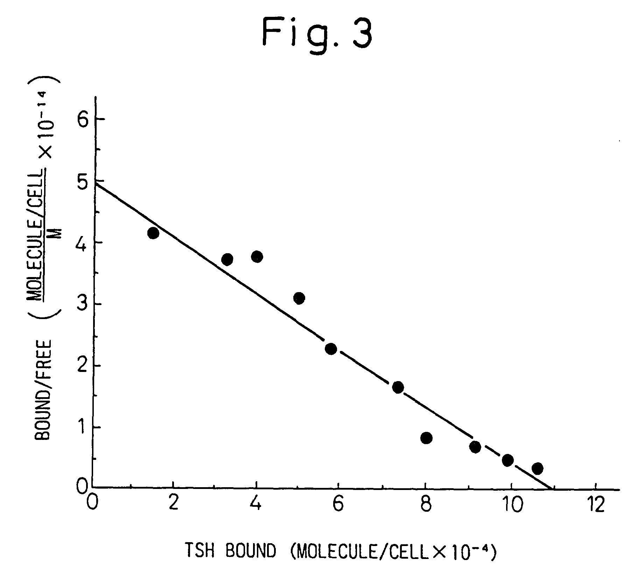

Fig. 3 is a Scatchard plot, with amount of bound labeled TSH plotted on the x-axis and the ratio of bound labeled TSH to free labeled TSH plotted on the y-axis.

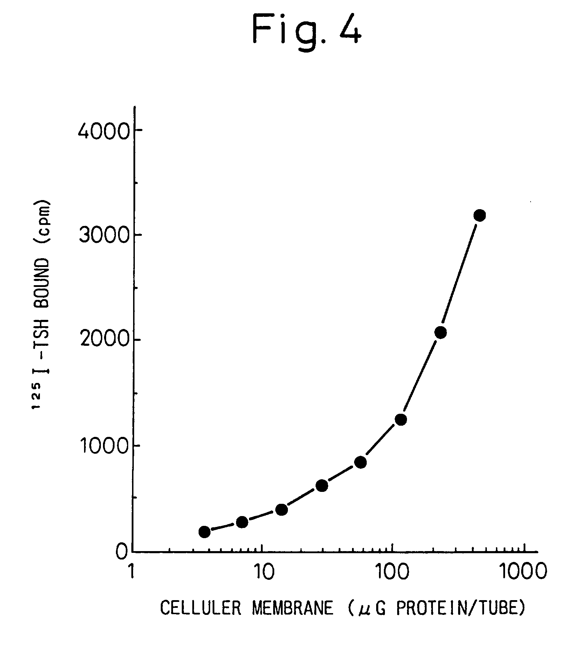

Fig. 4 is a graph showing the change in amount of bound labeled TSH with respect to the membrane fraction (SP56 membrane fraction) containing recombinant TSHR prepared according to the present invention. The Y-axis indicates the amount of SP56 membrane fraction and the X-axis indicates the amount of bound labeled TSH.

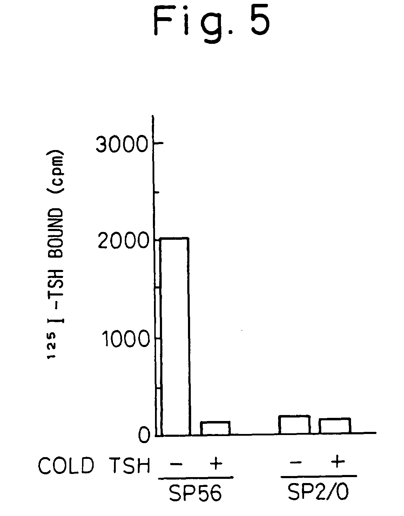

Fig. 5 is a graph showing inhibition of labeled TSH binding by unlabeled TSH, using the membrane fraction (SP56 membrane fraction) containing recombinant TSHR prepared according to the present invention. In this graph, "+" denotes the amount of bound labeled TSH in the presence of unlabeled TSH, and "-" denotes the amount of bound labeled TSH in the absence of unlabeled TSH. SP2/0 indicates the results for a membrane fraction prepared from recombinant TSHR non-expressing SP2/0 myeloma cells which were used as a control.

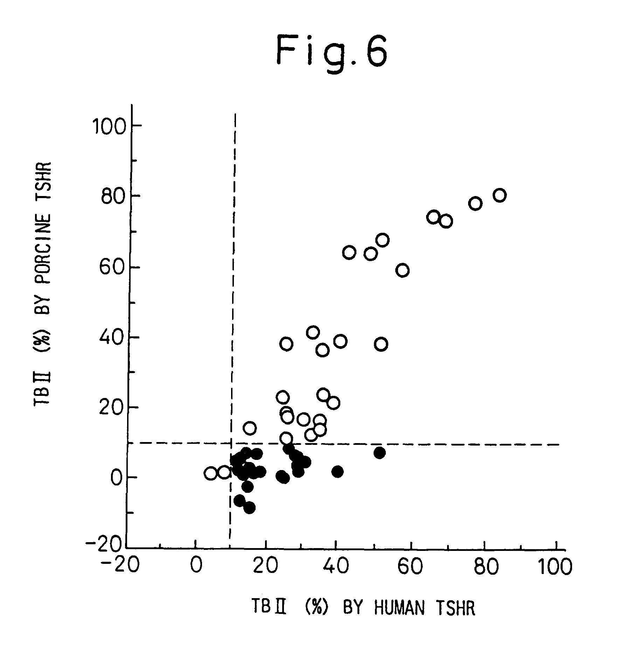

Fig. 6 is a graph showing the correlation between the results of assaying anti-TSHR autoantibody in samples, upon using a commercially available TBII (thyrotropin-binding inhibit immunoglobulin) assay kit and using a membrane fraction (SP56 membrane fraction) containing recombinant TSHR prepared according to the present invention. The x-axis indicates the results obtained using the SP56 membrane fraction and the y-axis indicates the results obtained with the commercially available assay kit.

In this graph, black circles are used to denote the sera which, of the sera judged to be negative with the commercially available kit, were judged as positive with the SP56 membrane fraction containing recombinant TSHR prepared according to the invention, and white circles are used to denote those which were judged as being either positive or negative by both assays. Judgment of positivity or negativity with the commercially available kit was made according to the cutoff value (dotted line parallel to the x-axis) given in the manual included with the kit. The dotted line parallel to the y-axis is the cutoff value determined based on the results of measuring 20 healthy persons.

### DETAILED DESCRIPTION

A more detailed description of the invention will now be given.

### 1. Myeloma cell line expressing recombinant TSHR

A myeloma cell line expressing recombinant TSHR according to the present invention may be prepared by a conventional genetic engineering method, i.e. by introducing one of the expression vectors mentioned below into a myeloma cell line.

A replicable expression vector to be used according to the present invention, which expresses the DNA sequence coding for membrane TSHR, i.e. which is capable of producing membrane TSHR, may be appropriately selected without any limitation so long as it contains the already reported DNA sequence coding for TSHR, in addition to the DNA sequences necessary for expressing that DNA sequence and the replication origin, etc. for replicating the vector DNA in the host, and is capable of transforming the designated host. An important DNA sequence necessary for expressing the desired DNA sequence is the promoter system, examples of which include the SV40 promoter system and the adenovirus promoter system, and it may be appropriately selected in consideration of the relationship with the host. Examples of combinations are SV40 promoter system/CHO cells (CHO-K1) and SV40 promoter system/dhfr-deficient CHO cells.

The DNA sequence coding for TSHR may be prepared with reference to existing literature (for example, BBRC, Vol.165, p.1184, 1989).

A preferred example of a myeloma cell line to be used according to the invention is the mouse myeloma cell line SP2/O (non-Ig-secreting mouse myeloma, CRL1581: ATCC) indicated in the Examples.

The transformation of the myeloma cell line with the expression vector may be carried out according to a conventional method such as electroporation, with no particular restrictions.

The culturing of the myeloma cells may be carried out by a conventional method. Specific examples include static culturing using a conventional incubator and mass culturing using a suitable cell culturing apparatus. The cell culturing apparatus may be a hollow fiber type or a fermentor type, but a fermentor type is preferred since it does not require the procedure of releasing the cells from a carrier.

The DNA sequence coding for TSHR which is introduced into the myeloma cells is expressed by the action of the promoter system, resulting in myeloma cells which express TSHR on their membrane. The membrane fraction may be prepared from the myeloma cells by a method in which, for example, the cells are homogenized with a homogenizer and the centrifugation precipitate from subsequent is recovered (Lectures on New Biochemical Experiments 9, Hormones I, Peptide Hormones, p.369, Tokyo Kagaku Dojin). A membrane fraction prepared in this manner contains recombinant TSHR.

### 2. Immunochemical assay method for anti-TSHR autoantibody

The object of the immunochemical assay method for anti-TSHR autoantibody according to the present invention is that of accurate and rapid measurement of the physiological concentration of anti-TSHR autoantibody contained in human serum.

A specific example of a measurement according to the invention is one wherein anti-TSHR autoantibody in a sample to be analyzed is allowed to bind with the aforementioned membrane fraction which is bound to a solid carrier, and the amount of bound anti-TSHR autoantibody is then measured.

In this case, the solid carrier used may be plate-shaped such as a microtiter plate, or it may be in the form of beads made of an organic substance, for example a metal ceramic or a plastic such as polystyrene or polypropylene.

Two possible methods for producing a solid phase of TSHR are a method of directly coating the membrane fraction of the membrane TSHR-expressing cells, and a method of binding it via anti-TSHR antibody. An example of a direct coating method is one in which a membrane fraction with a protein concentration of, for example, 1 mg/ml is added to the wells of a plate in an appropriate amount (for example, about 100 µl) and allowed to stand overnight. An example of a method of binding via anti-TSHR antibody is one in which the anti-TSHR antibody is first dissolved in a PBS solution to an appropriate concentration (for example, about 2 µg/ml) and then added to the wells of a plate in an appropriate amount (for example, about 100 µl) and allowed to stand for about one night, after which a membrane fraction with a protein concentration of, for example, about 1 mg/ml is added in an appropriate amount (for example, about 100 µl) and allowed to stand overnight.

The anti-TSHR autoantibody bound to a solid carrier via TSHR in this manner may itself be measured by a well known method such as the sandwich method or the competitive method. That is, in the case of the sandwich method, labeled anti-human immunoglobulin antibody may be used for the detection, and the anti-TSHR autoantibody bound to the solid carrier via the TSHR may be specifically detected by the labeled anti-human immunoglobulin antibody. Alternatively, in the case of the competitive method, labeled TSH or previously prepared labeled anti-TSHR antibody may be used. When labeled TSH is used, binding to TSHR by the labeled TSH and by the anti-TSHR autoantibody takes place in a competitive manner. The TSH used here may be bovine-derived or from any other non-human source, but human-derived TSH or TSH identical thereto such as recombinant human TSH is particularly preferred for use. When previously prepared labeled anti-TSHR antibody is used, binding to TSHR by the labeled anti-TSHR autoantibody and by the human serum likewise occurs in a competitive manner.

Accordingly, the present invention provides a method for assaying anti-human thyroid stimulating hormone receptor autoantibody comprising the steps of:
(1) immobilizing a membrane fraction, containing recombinant human thyroid stimulating hormone receptor produced by culturing a myeloma cell line which expresses the recombinant human thyroid stimulating hormone receptor, on a solid support;
(2) bringing a sample into contact with the solid support on which said membrane fraction has been immobilized;
(3) bringing a labeled anti-human immunoglobulin antibody into contact with the reaction mixture of the step (2); and
(4) measuring the label captured on said solid support.

The present invention further provides a method for assaying anti-human thyroid stimulating hormone receptor autoantibody, comprising the steps of:
(1) immobilizing a membrane fraction containing recombinant human thyroid stimulating hormone receptor produced by culturing a myeloma cell line which expresses the recombinant human thyroid stimulating hormone receptor, on a solid support;
(2) bringing a sample and a labeled thyroid stimulating hormone into contact with the solid support on which said membrane fraction has been immobilized; and
(3) measuring the label captured on said solid support.

The present invention still further provides a method for assaying anti-human thyroid stimulating hormone receptor autoantibody, comprising the steps of:
(1) immobilizing a membrane fraction containing recombinant human thyroid stimulating hormone receptor produced by culturing a myeloma cell line which expresses the recombinant human thyroid stimulating hormone receptor, on a solid support;
(2) bringing a sample and a labeled anti-human thyroid stimulating hormone receptor antibody into contact with the solid support on which said membrane fraction has been immobilized ; and
(3) measuring the label captured on said solid support.

The labelling to be used according to the present invention may be, for example, a radioactive substance, a fluorescent substance, a luminescent substance, an enzyme such as alkaline phosphatase or horseradish peroxidase, or biotin, but from the viewpoint of ease of handling an enzyme such as alkaline phosphatase or horseradish peroxidase is particularly preferred.

A reagent kit for assaying anti-TSHR autoantibody which is provided by the invention includes at least a membrane fraction containing recombinant TSHR produced by culturing a myeloma cell line which expresses recombinant TSHR, and it may also include other reagents necessary for the sandwich assay method or competitive assay method as described above.

### 3. Production of anti-TSHR monoclonal antibodies

A process for production of an anti-TSHR monoclonal antibody according to the present invention is characterized by immunizing a mouse with an immunogen, i.e., a myeloma cell line which expresses the present human TSHR. Since the present myeloma cell line is that prepared by transforming SP2/O cell line, which is widely used as a partner of fusion for mouse spleen cells, with an expression vector, then when a mouse such as Balb/c mouse is immunized by the cell line of the present invention only the human SHR is a heterologous protein for the Balb/c mouse, and therefore anti-TSHR monoclonal antibody can be produced. Examples of a screening method include the use of a cell sorter, and the use of a microtiter plate. In any screening method, the specific binding and non-specific binding can be distinguished by using a combination of SP2/O cells transformed with a TSHR expression vector and control SP2/O cells, or a combination of human TSHR-expressing CHO cells and control CHO cells.

The anti-TSHR monoclonal antibody thus obtained can be used for an immunological assay of anti-TSHR autoantibodies as described above.

### EXAMPLES

The present invention will now be explained in further detail by way of the following examples, which are in no way intended to restrict the invention.

### Example 1. Isolation of human TSHR gene

The series of gene recombination procedures described in this example (extraction of mRNA, cleavage of DNA with restriction endonuclease, etc.) were carried out according to the method of Maniatis, et al. (see Molecular Cloning, Cold Spring Harbor Laboratory, 1982) and Harwin, et al. (see DNA Cloning, A Practical Approach, Vol.1, p.49, Oxford, 1985). Screening of the λ-gtll cDNA library with antibodies was conducted using a commercially available vector (Lambda Directional Cloning Vector, product of Pharmacia Co.).

First, mRNA was extracted from human thyroid cells (thyroidectomized tissue), and λ-gtll (Clonetech Co.) was used to prepare a cDNA library. Next, the human-derived anti-TSHR monoclonal antibody TRMo (J.B.C., Vol.263, p.16341, 1988) was used to screen approximately 500,000 clones, and finally one clone was obtained. The cDNA of this clone was analyzed and found to be identical to the sequence reported by Rapoport, et al. (B.B.R.C., Vol.165, p.1184, 1989).

### Example 2. Construction of membrane TSHR gene-expressing vector

A recombinant human membrane TSHR gene-expressing vector was constructed using the TSHR gene obtained in Example 1 as the starting material.

An EcoRI fragment containing the entire TSHR gene was inserted into a commercially available phagemid vector pUC118 (product of Takara Shuzo, KK.) to obtain p118-TSHR. The EcoRI site was reintroduced into p118-TSHR just before the initiation codon using the oligonucleotide 5'-CCGCCGGCCTCATGAATTCCACGGGAC-3' and a commercially available kit (In Vitro Mutagenesis Kit, product of Amersham Co.).

After the EcoRI fragment (about 2.3 kbp) was first introduced into a commercially available vector (pBluescript, product of Toyobo, KK.), a fragment cut out with XhoI and NotI was inserted into the animal cell expression vector BCMGS neo (Eur. J. Immunol. 18, 97-104, 1988 and J. Exp. Med., 169, 13-25, 1989) to obtain the membrane TSHR gene-expressing vector pBCMGS-TSHR. The structure of this vector is shown in Fig. 1.

### Example 3. Establishment and selection of membrane TSHR-expressing myeloma cell line

The plasmid pBCMGS-hTSHR (10 µg) obtained in Example 2 was introduced by electroporation (Gene Pulser: 900 V, 25 µF, product of Biorad Co.) into 2 × 10⁷ SP2/0 cells (non-Ig-secreting mouse myeloma: CRL1581; ATCC). After standing on ice for 10 minutes, the cells were collected and cultured for 24 hours in E-RDF medium (containing 10% FCS). After further culturing for 3 hours in a selection medium (E-RDF medium, 10% FCS, 600 µg/mB G418), monoclonal cells were obtained by limiting dilution. The binding capacity of each of the clones to labeled TSH was evaluated by the method indicated in Example 7. Also, to confirm whether the binding to the labeled TSH was specific, a large excess (10⁻⁶M) of unlabeled TSH was added to 5 of the clones in which binding to labeled TSH was confirmed, to determine whether binding of the labeled TSH was inhibited. The results are shown in Fig. 2.

The results demonstrate that the binding of the labeled TSH was completely inhibited by addition of the unlabeled TSH, indicating specific binding of the labeled TSH to TSHR on the cell surfaces. The clone with the greatest amount of TSH binding (SP56) was selected as the membrane TSHR-expressing myeloma cell line.

The cell line SP56 was deposited with the National Institute of Bioscience and Human-Technology Agency of Industrial Science and Technology, 1-3, Higashi 1-chome, Tsukuba-shi, Ibaraki-ken 305 Japan, as an international deposition under the Budapest Treaty, as FERM BP-5349 on December 21 , 1995.

### Example 4. Preparation of SP56 cell membrane fraction

About 4 × 10⁸ SP56 cells were suspended in 20 ml of a buffer solution (15 mM Tris-HCl buffer solution, pH 7.5, 2 mM MgCl₂, 0.3 mM EDTA, 1 mM EGTA, 1 mM PMSF), and allowed to stand on ice for 5 minutes. The cells were then homogenized with a Teflon homogenizer. The resulting homogenate was centrifuged (15,000 × g, 15 minutes) and the pellet was collected. The collected pellet was suspended in 10 ml of an assay buffer solution included in a TBII assay kit (Cosmic Co.).

### Example 5. Mass suspension culturing of SP56 cells

Mass culturing of SP56 was attempted using a fermenter-type mass culturing apparatus (Axellon, product of Kirin Beer Co.). The SP56 cells (6 × 10⁸) were seeded in 3 L of E-RDF medium (containing 600 µg/mol G418 and 10% FCS) (1 × 10⁵ cells/ml). The cells were cultured for 3 days, and perfusion of the medium (3 L/day) was initiated when the cell count reached 1 × 10⁶ cells/ml. After an additional 3 days of perfusion culturing, the cell count reached 1 × 10⁷ cells/ml. The culturing was controlled to a temperature of 37°C, a pH of 7.5 and a dissolved oxygen content of 5 ppm.

After completion of the culturing, the cells were separated from the culture medium by centrifugation. Finally, 3 × 10¹⁰ cells were isolated from the 3 L fermenter. A membrane fraction was prepared from the obtained cells by the method indicated in Example 4, and the isolated membrane fraction had a protein content of about 3450 mg. This amount of the membrane fraction was enough to analyse about 15,000 samples. That is, using SP56 made it possible to easily prepare a membrane fraction necessary for assay for a substantial number of TBII samples.

### Example 6. TSH ¹²⁵I-labelling reaction

To a solution of 5 µg of bovine TSH (specific activity: 20 U/mg) dissolved in 5 µl of 0.1 M borate buffer pH 8.5, there was added a Bolton-Hunter reagent (148 TBq/mmol, product of Amersham Co.) dried with nitrogen gas, to begin labelling of the TSH. After a 15 minute reaction on ice, 0.5 ml of a 0.1 M borate buffer solution at pH 8.5 containing 0.2 M glycine was added to decompose the unreacted reagent. Gel filtration (G3000SWXL, product of TOSOH CORP.) was then performed to purify the labeled TSH.

### Example 7. Assay of TSH binding to SP2/0 cells

SP2/0 cells were centrifuged and separated from the medium. The obtained cells were washed once with modified Hank's buffer solution (280 mM sucrose added instead of NaCl to make an isotonic solution). The labeled TSH was added thereto at 8000 cpm and allowed to react at 37°C for one hour. This was followed by centrifugation and collection of the cells, washing of the cells with modified Hank's buffer solution and further centrifugation and collection of the cells. The radioactivity of the cells was then measured with a γ-counter. For measurement of the number of TSHR molecules expressed per cell, the amount of labeled TSH added was varied from 650 to 37,000 cpm, and the amount of bound labeled TSH was measured. As a result of a Scatchard plot based on the obtained numerical values, it was found that 1.1 × 10⁵ TSHR molecules had been expressed per cell, and that the Kd (dissociation constant) with bovine TSH was 2.2 × 10⁻¹⁰. The Scatchard plot obtained in this example is shown in Fig. 3.

### Example 8. Study of assay conditions using SP56 cell membrane fraction

Measurement was made of the change in the amount of bound labeled TSH upon variation of the amount of the SP56 cell membrane fraction obtained in Example 4. The results are shown in Fig. 4.

Based on Fig. 4, it was decided to use 230 µg of TSHR-containing fractions for a single measurement. Then, in order to confirm that the binding obtained here was specific binding between the labeled TSH and the TSHR in the cell membrane fraction, a large excess (10⁻⁶M) of unlabeled TSH was added to determine whether binding of the labeled TSH was inhibited. The results are shown in Fig. 5.

The results demonstrated that the binding of the labeled TSH was completely inhibited by addition of the unlabeled TSH. Thus it was clear that the labeled TSH had specifically bound to TSHR in the cell membrane fraction, and that this assay method may be useful for assay of autoantibody against TSHR.

### Example 9. TBII assay using SP56 cell membrane fraction

An evaluation was made of 53 serum samples from Basedow's disease patients. First, a commercially available TBII assay kit (TRAb Kit, product of Cosmic Co.) was used to measure TBII in the 53 samples. Membrane fraction in the amount indicated in Example 8 was then used to measure TBII in the specimens. The correlation between the TBII values obtained by each assay method is shown in Fig. 6.

The results showed a correlation of r=0.726. Of the 28 samples judged to be negative (points lower than 10% on the y-axis) by the conventional kit, 25 samples were judged to be positive (black circles in Fig. 6) by the present method using the SP56 membrane fraction. This indicates that TBII assay using the SP56 membrane fraction is more highly sensitive than the conventional TBII assay, and that using human TSHR normally recognized by anti-TSHR autoantibody in serum is more suitable for TBII assay systems than using porcine thyroid membrane fractions which contain porcine TSHR.

According to the present invention, it is possible to produce, in large quantities, recombinant human TSHR (recombinant TSHR) which is effective for the diagnosis of autoantibody diseases. A variety of additional effects may be expected for the present invention whereby the DNA sequence coding for TSHR is expressed in myeloma cells. That is, the use of myeloma cells allows expression of large quantities of TSHR. Also, since the myeloma cells are not adherent cells they may be cultured in suspension, thus allowing a relatively easy procedure, and allowing the culturing to be performed with a more simple apparatus, without the need for special skills, and in greater amounts than with adherent cells. Furthermore, since the myeloma cells are suspended cells, treatment with a protease such as trypsin is not necessary for recovery of the expressed TSHR and thus it may be recovered without causing cleavage of the TSHR which has membrane spanning regions. Hence, it becomes possible to produce TSHR which is more reliable, in terms of binding capacity with anti-TSHR autoantibody, than TSHR which has sustained damage by protease treatment.

As demonstrated by the examples given above, TSHR produced according to the present invention provides a more highly accurate assay than porcine TSHR which has conventionally been used to assay anti-TSHR autoantibody.

## Claims

1. A myeloma cell line expressing recombinant human thyroid stimulating hormone receptor.

2. A myeloma cell line according to claim 1, wherein the myeloma cell line is SP56 (FERM BP-5349).

3. A method for producing a membrane fraction containing recombinant human thyroid stimulating hormone receptor, comprises the step of culturing a myeloma cell line which expresses recombinant human thyroid stimulating hormone receptor.

4. A method according to claim 1, wherein the myeloma cell line is SP56 (FERM BP-5349).

5. A method for assaying anti-human thyroid stimulating hormone receptor autoantibody, comprising the step of bringing a sample to be analyzed into contact with a membrane fraction containing recombinant human thyroid stimulating hormone receptor produced by culturing a myeloma cell line which expresses the recombinant human thyroid stimulating hormone receptor.

6. A method according to claim 5, wherein the myeloma cell line is SP56 (FERM BP-5349).

7. A method for assaying anti-human thyroid stimulating hormone receptor autoantibody, comprising the steps of:
(1) immobilizing a membrane fraction containing recombinant human thyroid stimulating hormone receptor produced by culturing a myeloma cell line which expresses the recombinant human thyroid stimulating hormone receptor, on a solid support;
(2) bringing a sample into contact with the solid support on which said membrane fraction has been immobilized;
(3) bringing a labeled anti-human immunoglobulin antibody into contact with the reaction mixture of the step (2); and
(4) measuring the label captured on said solid support.

8. A method according to claim 7, wherein the myeloma cell line is SP56.(FERM BP-5349).

9. A method for assaying anti-human thyroid stimulating hormone receptor antoantibody, comprising the steps of:
(1) immobilizing a membrane fraction containing recombinant human thyroid stimulating hormone receptor produced by culturing a myeloma cell line which expresses the recombinant human thyroid stimulating hormone receptor on a solid support;
(2) bringing a sample and a labeled thyroid stimulating hormone into contact with the solid support on which said membrane fraction has been immobilized; and
(3) measuring the label captured on said solid support.

10. A method according to claim 9, wherein the myeloma cell line is SP56 (FERM BP-5349).

11. A method for assaying anti-human thyroid stimulating hormone receptor antoantibody, comprising the steps of:
(1) immobilizing a membrane fraction containing recombinant human thyroid stimulating hormone receptor produced by culturing a myeloma cell line which expresses the recombinant human thyroid stimulating hormone receptor on a solid support;
(2) bringing a sample and a labeled anti-human thyroid stimulating hormone receptor antibody into contact with the solid support on which said membrane fraction has been immobilized; and
(3) measuring the label captured on said solid support.

12. A method according to claim 11, wherein the myeloma cell line is SP56 (FERM BP-5349)

13. An assay kit for the assay of anti-human thyroid stimulating hormone receptor autoantibody, comprising at least a membrane fraction containing recombinant human thyroid stimulating hormone receptor produced by culturing a myeloma cell line which expresses recombinant human thyroid stimulating hormone receptor.

14. An assay kit according to claim 13, wherein the myeloma cell line is SP56 (FERM BP-5349).

15. A process for obtaining an anti-human thyroid stimulating hormone receptor monoclonal antibody, comprising the step of immunizing an animal with a myeloma cell-line expressing a recombinant human thyroid stimulating hormone receptor.

16. A process according to claim 15, wherein the myeloma cell line is SP56 (FERM BP-5349).

17. A kit for measurement of anti-human thyroid stimulating hormone receptor autoantibody, comprising a solid support to which a recombinant or naturally occurring thyroid stimulating hormone receptor has been immobilized using an anti-human thyroid stimulating hormone receptor monoclonal antibody obtained by a process according to claim 15.

18. A kit for measurement of anti-human thyroid stimulating hormone receptor autoantibody, comprising a solid support to which a recombinant or naturally occurring thyroid stimulating hormone receptor has been immobilized using an anti-human thyroid stimulating hormone receptor monoclonal antibody obtained by a process according to claim 18.
